(19) Europäisches Patentamt
European Patent Office
Office européen des brevets

(11) Publication number: **0 175 323 B1**

# (12) EUROPEAN PATENT SPECIFICATION

(45) Date of publication of patent specification: **29.04.92**

(51) Int. Cl.5: **C07K 5/00**, C07K 7/02, C07K 7/06, A61K 37/02

(21) Application number: **85111688.9**

(22) Date of filing: **16.09.85**

## (54) Biologically active peptides, processes for preparing them and pharmaceutical compositions.

(30) Priority: **20.09.84 GB 8423771**
**18.06.85 US 745932**

(43) Date of publication of application:
**26.03.86 Bulletin 86/13**

(45) Publication of the grant of the patent:
**29.04.92 Bulletin 92/18**

(84) Designated Contracting States:
**AT BE CH DE FR GB IT LI LU NL SE**

(56) References cited:
**EP-A- 0 025 218**
**EP-A- 0 083 849**
**FR-A- 2 465 713**

**CHEMICAL ABSTRACTS, vol. 100, no. 3, 16th January 1984, page 68, abstract no. 17851j, Columbus, Ohio, US; A.W. LIPKOWSKI et al.: "Double opiate peptides. A hypothesis of two different mechanisms of opiate actions" & PEPT., PROC. EUR. PEPT. SYMP., 17th 1982 (PUB. 1983), 481-6**

(73) Proprietor: **FARMITALIA CARLO ERBA S.r.L.**
**Via Carlo Imbonati 24**
**I-20159 Milano(IT)**

(72) Inventor: **de Castiglione, Roberto**
**Via Domenichino 38**
**I-20 149 Milan(IT)**
Inventor: **Perseo, Giuseppe**
**Via Monte Bianco, 66**
**I-20 033 Desio (Milan)(IT)**
Inventor: **Mena, Renzo**
**Via Caduti, 1**
**I-28 066 Galliate (Novara)(IT)**
Inventor: **Cervini, Maria Antonietta**
**Via Nazario Sauro, 10**
**I-21 010 Cardano Al Campo (Varese)(IT)**
Inventor: **Rossi, Alessandro**
**Via L. Barzini, 7**
**I-20 125 Milan(IT)**

(74) Representative: **Lehn, Werner, Dipl.-Ing. et al**
**Hoffmann, Eitle & Partner Patentanwälte Arabellastrasse 4**
**W-8000 München 81(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(-/2.17/2.0)

CHEMICAL ABSTRACTS, vol. 101, no. 21, 19th November 1984, page 90, abstract no. 184200y, Columbus, Ohio, US; R. DE CASTIGLIONE: "Structure-activity relationships in demorphin-like peptides" & HIGHLIGHTS RECEPT. CHEM., PROC. CAMERINO SYMP. RECENT ADV. RECEPT. CHEM., 2ND 1983 (PUB. 1984), 149-68

## Description

The present invention relates to biologically active peptides, their pharmaceutically acceptable salts, processes for their preparation and their application as therapeutic agents,namely for the treatment of addictive behavior and opiate- and alcohol-dependence.

Opiate- and alcohol-dependence constitutes one of the major problems plaguing modern civilization. In an attempt to curb the desire for alcohol or opiates by persons addicted to such substances, a variety of chemical substances have been investigated. However, while some of these substances have the ability to lessen the desire for alcohol or opiates, many of these substances are, themselves, addictive and cause the recipient to experience withdrawal symptoms at the cessation of administration.

Accordingly, a need continues to exist for substances which can curb the desire of alcohol- or opiate-dependent mammals, particularly humans, for alcohol or opiates, without eliciting addictive behavior, themselves.

Peptides 1982, pages 481-486 discloses that synthesized double peptides of formula $(Tyr\text{-}X\text{-}Phe\text{-}NH\text{-})_2$ have opiate agonist and antagonist activities; where X represents D-Ala or D-Thr.

EP-A-0083849 discloses that dimers of peptide amides including the bridging group $-NH-(CH_2)_n-NH-$ act as selective delta-opiate-receptor antagonists.

Highlights in Receptor Chemistry, pages 149-168, (1983) disclose various dermorphin-like peptides which include a D-Ala moiety as an essential moiety. Peptides are disclosed having opiate-like activity which include the sequence "Tyr-D-Ala-Phe-".

Accordingly, it is an object of the present invention to provide peptides and compositions containing them which are able to curb the desire of alcohol- or opiate-dependent mammals for alcohol or opiates.

It is also an object of this invention to provide peptides and compositions containing them which are able to curb the desire of alcohol- or opiate-dependent mammals for alcohol or opiates without eliciting addictive behavior, themselves.

Further, it is also an object of the present invention to provide a method of preparing the peptides of the formula I as herein under defined.

According to the present invention, peptides are now provided having the formula (I) :

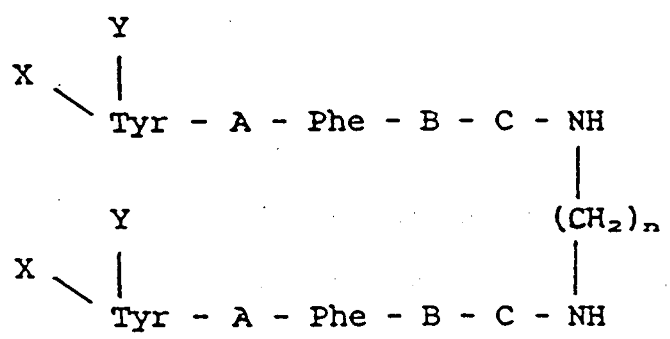

I

wherein

- X represents a hydrogen atom or a t-butyloxy carbonyl group,
- Y represents a hydrogen atom or a benzyl group
- A represents D-Ala or D-Arg residue,
- B represents a glycine, D-Ala or Sar residue,
- C represents a valence bond or Tyr, Tyr-Pro-Ser residue and
- n is 0, 2, 4 or 12; or the pharmaceutically acceptable salts thereof.

Salts of peptides according to the invention with pharmaceutically acceptable salts are within the scope of the invention. Such acid addition salts can be derived from a variety of inorganic and organic acids such as sulfuric, phosphoric, hydrochloric, hydrobromic, hydroiodic, nitric, sulfamic, citric, lactic, pyruvic, oxalic, maleic, succinic, tartaric, cinnamic, acetic, trifluoroacetic, benzoic, salicylic, gluconic, ascorbic and related acids.

The process for preparing the peptides of the present invention is characterized by condensing a peptide of the formula II

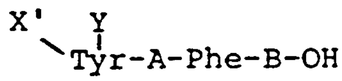

II

wherein Y, A and B are as defined above, and X' has the same meanings of X stated above but not hydrogen atom, with a compound of the formula III

$[H-C-NH-(CH_2)_n]_2$ III

wherein C and n are as defined above, using as activating method mixed anhydride, azide, activated ester or dicyclohexylcarbodiimide, optionally in the presence of a racemization preventing agent, such as N-hydroxysuccinimide or 1-hydroxybenzotriazole. The condensation may be carried out in a solvent such as dimethylformamide, dimethylacetamide, pyridine, acetonitrile, tetrahydrofuran or N-methyl-2-pyrrolidone. The reaction temperature may be from -30° C to ambient temperature. The reaction time is generally from 1 to 120 hours. The scheme of synthesis, the protecting groups and the condensing agents are selected so as to avoid the risk of racemization.

The starting compounds of the formula II and III are known derivatives or they may be prepared by classical solution method. The synthesis is carried out so that the resulting compounds of the formula II and III have the desired sequence of amino acid residues, and the connecting bridge of the appropiate length. The amino acids and peptides, which are condensed according to methods known in themselves in polypeptide chemistry, have their amino and carboxyl groups, which are not involved in the formation of the peptide linkage, blocked by a suitable protecting group. The protecting groups are capable of being removed by acid or alkali treatment and by hydrogenolysis. For the protection of the amino group the following protective groups may, for example, be employed: benzyloxycarbonyl, t-butoxycarbonyl, trityl, formyl, trifluoroacetyl, o-nitrophenylsulphenyl, 4-methoxybenzyloxycarbonyl, 9-fluorenylmethoxycarbonyl, 3,5-dimethoxy-α-α'-dimethylbenzyloxycarbonyl or methylsulphonylethoxycarbonyl.

For the protection of the carboxyl group; the following protective groups may, for example, be employed: methyl, ethyl, t-butyl, benzyl, p-nitrobenzyl or fluorenylmethyl.

The hydroxy functions of hydroxy amino acids may be protected by suitable protecting groups, throughout all the synthesis or only during a few steps, or may be kept unprotected. Deprotecting reactions are carried out according to methods known in polypeptide chemistry.

The condensation between an amino group of one molecule and a carboxyl group of another molecule to form the peptide linkage may be carried out through an activated acyl-derivative such as a mixed anhydride, an azide or an activated ester, or by direct condensation between a free amino group and a free carboxyl group, in the presence of a condensing agent such as dicyclohexylcarbodiimide, alone or together with a racemization preventing agent, such as N-hydroxysuccinimide or 1-hydroxybenzotriazole. The condensation may be carried out in a solvent such as dimethylformamide, dimethylacetamide, pyridine, acetonitrile, tetrahydrofuran or N-methyl-2-pyrrolidone. The reaction temperature may be from -30° C to ambient temperature. The reaction time is generally from 1 to 120 hours. The scheme of synthesis, the protecting groups and the condensing agents are selected so as to avoid the risk of racemization.

The compounds according to the invention exhibit a therapeutic utility for the treatment of addictive behaviour and alcohol-and opiate-dependence. These compounds are of particular interest as they are devoid of opiate activity in vivo after systemic administration. Moreover, these compounds have no addicting liability.

The studies were carried out on experimental animals as follows: Male rats were instrumentally conditioned to the i.c.v. self administration of morphine following the technique described by J.R. Weeks and R.G. Collins (Psychopharmacol. (1979) 65, 171-177), until spontaneously seeking for morphine-injections up to the 13th day. At this time, morphine seeking rats were treated i.c.v. (10 mcg/kg/h) or s.c. (30 mg/kg) with the compounds of the present invention. These treatments were surprisingly able to extinguish for several hours the opiate-seeking behaviour of the dependent animals.

Repeated administration of raising doses of the compounds were performed in mice and rats in order to exclude, after the discontinuation of treatments and/or after the administration of the opioid antagonist naloxone (10 mg/Kg, s.c.), the precipitation of signs of withdrawal. No signs of withdrawal syndrome were

ever observed, nor did the present compounds prove able to substitute for morphine when administered to morphine dependent rats. Dermorphin, an opiate-like heptapeptide was used as a standard compound. The pharmacological profiles of dermorphin,compound XV and compound X are set forth in the following table:

| | 1) Dermorphin | 2) Compound XV | 3) Compound X |
|---|---|---|---|
| Induction of opiate-seeking behaviour (OSB) (rat, i.c.v.) | + | - | - |
| Deconditioning of OSB (rat, i.c.v. and s.c.) | S.M. | ++ | +++ |
| Reduction of opiate withdrawal syndrome (rat, i.c.v. and s.c.) | ++ | + | n.d. |
| Reduction of ethanol withdrawal syndrome (mouse, s.c.) | n.d. | + | +++ |

S.M.: substitution for morphine

1) de Castiglione et al. Int. J. Peptide Protein Res. 17, 263-272 (1981)

2)

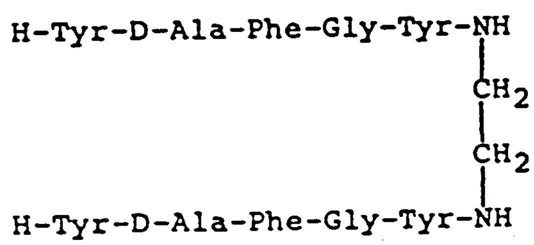

.2HCl

3)

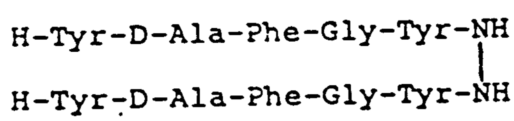

.2HCl

The compounds according to the invention have also been shown to exhibit a similar activity observed in studies in mice. The studies were carried out on experimental animals according to the procedure described in D.B. Goldstein in J. Pharmacol. Exp. Ther. 180, 203-215 (1972) and ibidem, 183, 14-22 (1972). A subcutaneous injection of 50 mg/kg of the compounds of the present invention was able to extinguish in a few hours the alcohol abstinence syndrome of the dependent animals. Recommended doses in humans by systemic route of administration (i.m., s.c., i.v. or sublingual) are in the range of about 5 to 100 mg x 2 to 4 times a day.

Preferred compounds according to the present invention are listed below:

1) 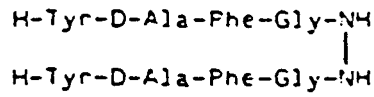

2) 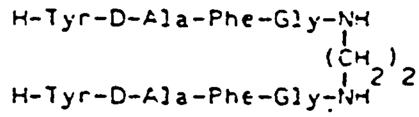

3) 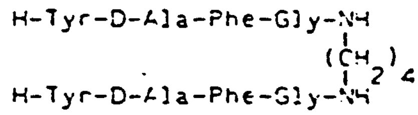

5) 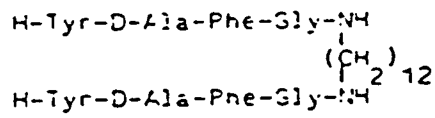

6) 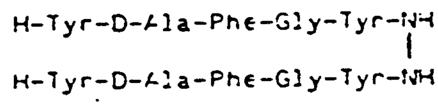

7) 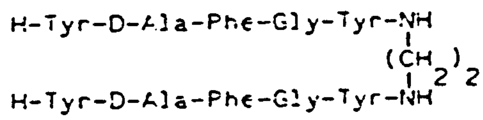

8) 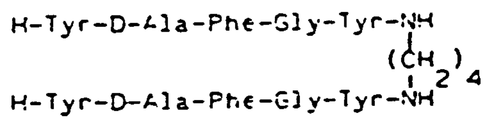

10) 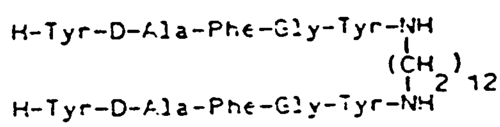

14) 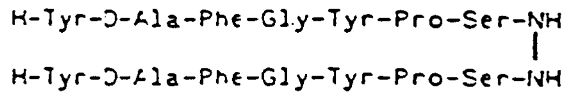

15) 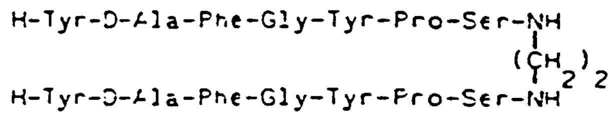

16) 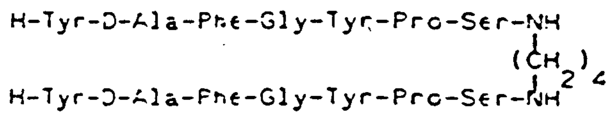

17) 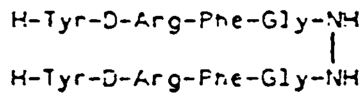

18) 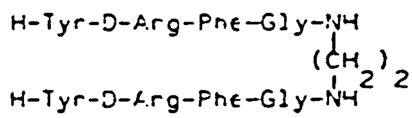

19) 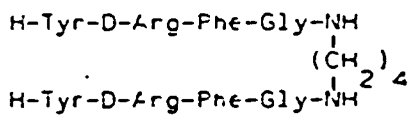

20) 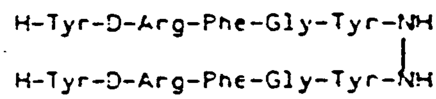

21) 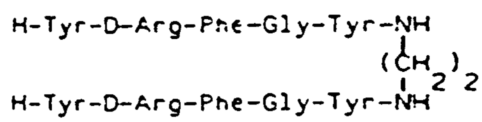

22) 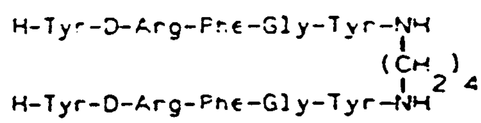

26) 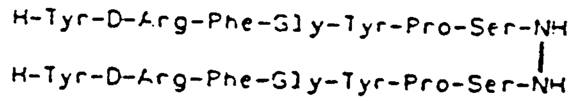

27) 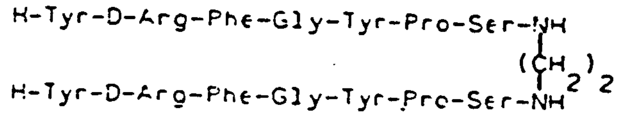

28) 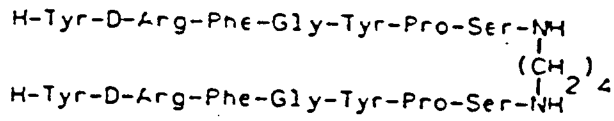

29) 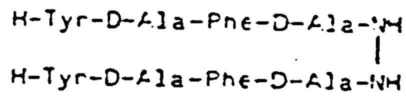

30) 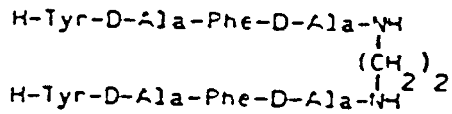

31) 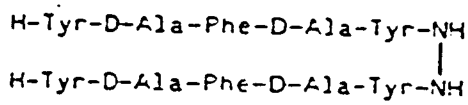

32) 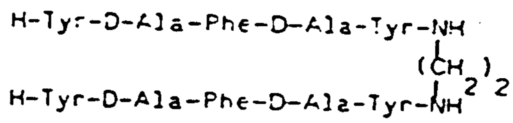

35) 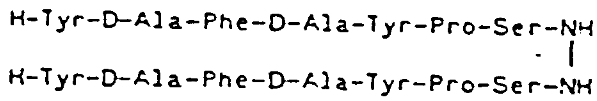

36) 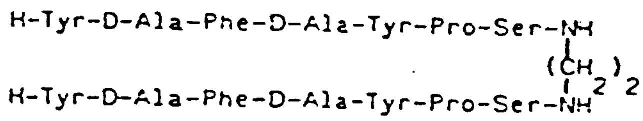

37) H-Tyr-D-Arg-Phe-D-Ala-NH
|
H-Tyr-D-Arg-Phe-D-Ala-NH

38) H-Tyr-D-Arg-Phe-D-Ala-NH
$(CH_2)_2$
H-Tyr-D-Arg-Phe-D-Ala-NH

39) H-Tyr-D-Arg-Phe-D-Ala-Tyr-NH
|
H-Tyr-D-Arg-Phe-D-Ala-Tyr-NH

40) H-Tyr-D-Arg-Phe-D-Ala-Tyr-NH
$(CH_2)_2$
H-Tyr-D-Arg-Phe-D-Ala-Tyr-NH

43) H-Tyr-D-Arg-Phe-D-Ala-Tyr-Pro-Ser-NH
|
H-Tyr-D-Arg-Phe-D-Ala-Tyr-Pro-Ser-NH

44) H-Tyr-D-Arg-Phe-D-Ala-Tyr-Pro-Ser-NH
$(CH_2)_2$
H-Tyr-D-Arg-Phe-D-Ala-Tyr-Pro-Ser-NH

55) H-Tyr-D-Arg-Phe-Sar-NH
|
H-Tyr-D-Arg-Phe-Sar-NH

56) H-Tyr-D-Arg-Phe-Sar-NH
$(CH_2)_2$
H-Tyr-D-Arg-Phe-Sar-NH

57) H-Tyr-D-Arg-Phe-Sar-Tyr-NH
|
H-Tyr-D-Arg-Phe-Sar-Tyr-NH

58) H-Tyr-D-Arg-Phe-Sar-Tyr-NH
$(CH_2)_2$
H-Tyr-D-Arg-Phe-Sar-Tyr-NH

61) H-Tyr-D-Arg-Phe-Sar-Tyr-Pro-Ser-NH
|
H-Tyr-D-Arg-Phe-Sar-Tyr-Pro-Ser-NH

62) H-Tyr-D-Arg-Phe-Sar-Tyr-Pro-Ser-NH
$(CH_2)_2$
H-Tyr-D-Arg-Phe-Sar-Tyr-Pro-Ser-NH

In this specification symbols and abbreviations are those commonly used in peptide chemistry (see Eur. J. Biochem. (1984) 138, 9-37). Other symbols and abbreviations used are: AcOH, acetic acid; AcOEt, ethyl acetate; n-BuOH, n-butyl alcohol; Bzl, benzyl alcohol, Cha, 3-(cyclohexyl)alanine; DCC, dicyclohexylcarbodiimide; DCEU, dicyclohexylurea; DMF, dimethylformamide; ECC, ethylchloroformate; FC, flash chromatography on silica gel (Merck) 0.040-0.063 mm; HCl/THF, dry HCl in anhydrous tetrahydrofuran; HOBt, 1-hydroxybenzotriazole; MeOH, methyl alcohol; NMM, N-methylmorpholine; PE, petroleum ether; Phg, 2-(phenyl)glycine; $iPr_2O$, diisopropyl ether; iPrOH, isopropyl alcohol; THF, tetrahydrofuran;

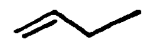 ,

Allyl group and Ppa, pipecolic acid.

For the sake of simplicity, in the experimental part a single line abbreviation for the peptides is used. In order to clarify this kind of abbreviation two examples are given here below:

a) [H-Tyr-D-Ala-Phe-Gly-NH-CH$_2$]$_2$ .2 HCl means

```
H-Tyr-D-Ala-Phe-Gly-NH
                    |
                  (CH2)2    .2 HCl
                    |
H-Tyr-D-Ala-Phe-Gly-NH
```

b) [Boc-Ser-NH]$_2$ means

```
Boc-Ser-NH
        |
Boc-Ser-NH
```

The following Examples will be seen to only illustrate the present invention without limiting the same. The $R_f$ values are determined on pre-coated plates of silica gel 60 $F_{254}$ (Merck), layer thickness 0.25 mm, length 20 cm, using the following development systems:

System A: benzene/benzine (60-80)/ethyl acetate = 70/10/40 by volume.
System B: benzene/ethyl acetate/acetic acid/water = 100/100/20/10 by volume (upper phase).
System C: benzene/ethyl acetate/acetic acid/water = 100/100/40/15 by volume (upper phase).
System D: n-butanol/acetic acid/water = 4/1/1 by volume.
System E: ethyl acetate/methanol = 9/1 by volume.
System F: n-butanol/acetic acid/water/pyridine = 4/1/1/0.125.
System G: chloroform/methanol = 8/2 by volume.

(Merck is a trade-mark.)

TLC analyses are carried out at a temperature ranging from 18 to 25 °C: the Rf values can therefore change ± 5%. Melting points are determined in open capillaries with a Tottoli apparatus and are uncorrected. Most of the derivatives soften and decompose before melting. High voltage paper electrophoresis is carried out with a Pherograph-Original-Franckfurt Type 64 apparatus on Schleicher and Schull paper No. 2317 at pH 1.2 (formic acid:acetic acid:water = 123:100:777) at 1600 V (40V/cm). The products are characterized by their mobilities relative to Glu at pH 1.2 ($E_{1.2}$). Optical rotations are measured photoelectrically with a Perkin-Elmer 141 polarimeter at a concentration c = 1 in methanol unless otherwise stated.

EXAMPLES

Example 1

Preparation of [H-Tyr-D-Ala-Phe-Gly-NH-$CH_2$}$_2$ . 2 HCl (II)

Step 1. [Boc-Tyr-D-Ala-Phe-Gly-NH-$CH_2$}$_2$ (I)

To a precooled solution (0° C) of 0.13 ml (2 mmol) of $NH_2$-$CH_2$-$CH_2$-$NH_2$, 2.23 g (4 mmol) of Boc-Tyr-D-Ala-Phe-Gly-OH (R. de Castiglione et al. (1981), Int. J. Peptide Protein Res. 17, 263) and 0.59 g (4 mmol) of HOBt in 30 ml of DMF, 0.91 g (4.4 mmol) of DCC were added. The mixture was allowed to react at 0° C for 4 hours, then at room temperature overnight. DCEU was removed by filtration and the solvent by evaporation in vacuo. The residue was dissolved in 20 ml of DMF and poured dropwise in 200 ml of a 10% citric acid aqueous solution cooled to 0° C. The product was filtered, washed to neutrality with water, dissolved in DMF and the resulting solution evaporated in vacuo. 1.64 g of partially purified compound I were obtained from iPrOH/$iPr_2O$/PE. The purification was increased by FC eluting with AcOEt:MeOH = 9:1 obtaining 1.39 g (61% yield) of compound I from iPrOH/$iPr_2O$. $Rf_C$ 0.30; $Rf_E$ 0.35; $Rf_G$ 0.81.

Step 2. [H-Tyr-D-Ala-Phe-Gly-NH-$CH_2$}$_2$ . 2HCl (II)

1.21 g (1.06 mmol) of [Boc-Tyr-D-Ala-Phe-Gly-NH-$CH_2$}$_2$ (I) were dissolved at room temperature in 12 ml of a 3.5 M solution of HCl/THF. After 30 minutes Boc-removal was complete and the solvents were evaporated in vacuo. The residue was dissolved in methanol and the solution was concentrated to dryness. 1.03 g (96% yield) of compound II were obtained from iPrOH/$iPr_2O$. $[\alpha]_D^{25}$ + 54.4°; $Rf_D$ 0.50; $E_{1.2}$ 0.82.

Example 2

Preparation of [H-Tyr-D-Ala-Phe-Gly-NH-$(CH_2)_2$}$_2$ . 2HCl (IV)

Step 1. [Boc-Tyr-D-Ala-Phe-Gly-NH-$(CH_2)_2$}$_2$ (III)

To a precooled solution (0° C) of 0.32 g (2 mmol) of 2 HCl . $NH_2$-$(CH_2)_4$-$NH_2$, 0.5 ml (4 mmol) of NMM, 2.23 g (4 mmol) of Boc-Tyr-D-Ala-Phe-Gly-OH (see Example 1. Step 1) and 0.59 g (4 mmol) of HOBt in 30 ml of DMF, 0.91 g (4.4 mmol) of DCC were added. The mixture was allowed to react at 0° C for 4 hours, then at room temperature overnight. DCEU was removed by filtration and the solvent evaporated in vacuo. The product was partially purified by pouring into an acidic solution as described for the compound I (Example 1, Step 1), obtaining 1.96 g (84% yield) of III from iPrOH/$iPr_2O$/PE and used as such in the next step. $Rf_C$ 0.26; $Rf_E$ 0.35; $Rf_G$ 0.81.

Step 2. [H-Tyr-D-Ala-Phe-Gly-NH-$(CH_2)_2$}$_2$ . 2 HCl (IV)

[Boc-Tyr-D-Ala-Phe-Gly-NH-$(CH_2)_2$}$_2$ (1.75 g, 1.50 mmol) (III) was deblocked as described in Example 1, Step 2. The crude product was successively purified by FC eluting with $CH_2Cl_2$:MeOH:$H_2O$ = 8:2:0.2 and by gel chromatography on Sephadex G-25 eluting with DMF. After restoring the anion with HCl/THF, 1.04 g (67% yield) of compound IV were obtained from iPrOH/$iPr_2O$. $[\alpha]_D^{26}$ + 49.5°; $Rf_D$ 0.47; $E_{1.2}$ 0.80 Glu.

Example 3

Preparation of [H-Tyr-D-Ala-Phe-Gly-NH-$(CH_2)_6$}$_2$ .2HCl (VI)

Step 1. [Boc-Tyr-D-Ala-Phe-Gly-NH-$(CH_2)_6$}$_2$ (V)

Boc-Tyr-D-Ala-Phe-Gly-OH (1.67 g, 3 mmol) (see Example 1, Step 1) and $H_2N$-$(CH_2)_{12}$-$NH_2$ (0.30 g, 1.5 mmol) were condensed as described in Example 2, Step 1 to give (from iPrOH/$iPr_2O$) 1.44 g (75% yield) of a partially purified compound V which was used as such in the next step; $Rf_c$ 0.57.
Step 2.[H-Tyr-D-Ala-Phe-Gly-NH-$(CH_2)$6 }$_2$ .2 HCl (VI) 1.3 g [Boc-Tyr-D-Ala-Phe-Gly-NH-$(CH_2)_6$}$_2$ (V) were dissolved in 13 ml of a 3.5 M solution of HCl/THF. After 30 minutes the Boc-removal was complete and the

solution was diluted with a great excess of diethyl ether obtaining the precipitation of the crude product. After purification accomplished by FC eluting with $CH_2Cl_2$:MeOH = 8:2; compound VI (0.65 g, 55% yield) was obtained from iPrOH/$iPr_2O$. $[\alpha]_D^{22}$ + 32.0; $Rf_F$ 0.75; $E_{1.2}$ 0.72.

Example 4

Preparation of [H-Tyr-D-Ala-Phe-Gly-Tyr-NH$\}_2$ . 2 HCl (X)

Step 1. [Boc-Tyr-NH$\}_2$ (VII)

To a solution of 1.09 g (3.87 mmol) of Boc-Tyr-OH in 15 ml of anhydrous THF, 0.43 ml (3.87 mmol) of NMM and 0.39 ml (3.87 mmol) of ECC were successively added at a temperature of -12 °C. After stirring at this temperature for 2 minutes, a cold solution of 1.14 g (3.87 mmol) of Boc-Tyr-NH-$NH_2$ (E. Schröder, (1963) Liebigs Ann. Chem. 670, 127) and 0.43 ml of NMM (3.87 mmol) in 15 ml of DMF was added. The reaction mixture was stirred for 45 minutes at -12 °C and for 90 minutes at 0-15 °C, then filtered from salts and evaporated in vacuo. The residue was dissolved in ethyl acetate and washed several times successively with sodium chloride saturated solutions of 1M citric acid, 1M sodium bicarbonate and water. The organic layer was dried over anhydrous sodium sulfate and the solvent removed in vacuo, obtaining a partially purified compound VII as an oil. The product was in a pure form (1.60 g, 74% yield) after a purification by FC eluting with $CHCl_3$:MeOH = 97:3. $[\alpha]_D^{24}$ - 14.4 °; $Rf_A$ 0.44

Step 2. [H-Tyr-NH$\}_2$ . 2 HCl (VIII)

1.45 g (2.60 mmol) of [Boc-Tyr-NH$\}_2$ (VII) were dissolved in 15 ml of a 3.5 M solution of HCl/THF. After 30 minutes Boc-removal was complete and the solution was diluted with a large amount of diethyl ether obtaining the precipitation of partially pure product VIII (1.01 g, 90% yield) that was used as such in the next step. $Rf_D$ 0.28; $E_{1.2}$ 1.47 Glu.

Step 3. [Boc-Tyr-D-Ala-Phe-Gly-Tyr-NH$\}_2$ (IX)

Boc-Tyr-D-Ala-Phe-Gly-OH (see Example 1, Step 1) (2.45 g, 4.4 mmol) and [H-Tyr-NH$\}_2$ . 2 HCl (VIII) (0.95 g, 2.2 mmol) were condensed as described in this Example, Step 1, but purifying the product as reported in Example 1, Step 1. 2.68 g (85% yield) of partially purified compound IX were obtained from iPrOH/$iPr_2O$/$Et_2O$. $Rf_B$ 0.10; $Rf_C$ 0.34; $Rf_G$ 0.66.

Step 4. [H-Tyr-D-Ala-Phe-Gly-Tyr-NH$\}_2$. 2 HCl

2.5 g (1.74 mmol) of [Boc-Tyr-D-Ala-Phe-Gly-Tyr-NH$\}_2$ (IX) were dissolved in a mixture of 25 ml of a 3.5 M solution of HCl/THF and 5 ml of formic acid. After 30 minutes Boc-removal was complete and solvents evaporated in vacuo obtaining the crude product X as an oil. After a purification accomplished by FC eluting with $CHCl_3$:MeOH = 7:3, 1.34 g (59% yield) of compound X were obtained from iPrOH/$iPr_2O$. $\{\alpha]_D^{21}$ + 65.9 °; $Rf_F$ 0.77: $E_{1.2}$ 0.69.

Example 5

Preparation of [H-Tyr-D-Ala-Phe-Gly-Tyr-NH-$CH_2\}_2$ •2 HCl (XV)

Step 1. [Boc-Tyr(Bzl)-NH-$CH_2\}_2$ (XI)

To a solution of 5.62 g (12 mmol) of Boc-Tyr(Bzl)-OH (C. Sorg et al, Liebigs Ann. Chem. (1970) 734, 180) in 60 ml of DMF, 0.41 ml (6 mmol) of $H_2N$-$CH_2$-$CH_2$-$NH_2$ were added and the mixture was allowed to react overnight. The solvent was removed in vacuo and the oily residue dissolved in ethyl acetate and washed several times successively with aqueous solutions of 1 M citric acid, 1M $NaHCO_3$ and sodium chloride saturated solution. The organic layer was dried over anhydrous sodium sulphate and the solvent removed in vacuo. 3.17 g (69% yield) of compound XI were obtained from iPrOH/$iPr_2O$. m.p. 206 °C; $[\alpha]_D^{24}$ - 4.6 ° (c = 1 DMF); $Rf_A$ 0.28; $Rf_B$ 0.75.

Step 2. [H-Tyr(Bzl)-NH-$CH_2$]$_2$ •2HCl (XII)

[Boc-Tyr(Bzl)-NH-$CH_2$]$_2$ (3.12 g, 4.07 mmol) (XI) was deblocked as described in Example 1, Step 2, to give 2.26 g (87% yield) of Compound XII from iPrOH/$iPr_2O$. $Rf_D$ 0.47; $E_{1.2}$ 1.24.

Step 3. [Boc-Tyr-D-Ala-Phe-Gly-Tyr(Bzl)-NH-$CH_2$]$_2$ (XIII)

Boc-Tyr-D-Ala-Phe-Gly-OH (3.74 g, 6.72 mmol) (see Example 1, Step 1) and [H-Tyr(Bzl)-NH-$CH_2$]$_2$ • 2 HCl (2.15 g, 3.36 mmol) (XII) were condensed as described in Example 1, Step 1 but with the addition of 0.12 g 0.12 g (1.01 mmol) of 4-dimethylamino-pyridine.

After the work up, accomplished as describe in the cited Example, 3.20 g (58% yield) of partially purified compound XIII were obtained from MeOH. $Rf_B$ 0.67, $Rf_C$ 0.70.

Step 4. [Boc-Tyr-D-Ala-Phe-Gly-Tyr-NH-$CH_2$]$_2$

3.02 g (1.84 mmol) of [Boc-Tyr-D-Ala-Phe-Gly-Tyr-(Bzl)-NH-$CH_2$]$_2$ (XIII) dissolved in 60 ml of DMF were hydrogenated at room temperature and atmospheric pressure in the presence of 1.51 g of 10% palladium-on-charcoal. The catalyst was removed by filtration and the solution was concentrated in vacuo. 1.97 g (73% yield) of partially pure compound XIV were obtained from iPrOH/$iPr_2O$; $Rf_C$ 0.26; $Rf_G$ 0.69.

Step 5. [H-Tyr-D-Ala-Phe-Gly-Tyr-NH-$CH_2$]$_2$

1.85 g (1.26 mmol) of [Boc-Tyr-D-Ala-Phe-Gly-Tyr-NH-$CH_2$]$_2$ (XIV) were dissolved in 20 ml of 3.5 M solution of HCl/THF. After 30 minutes the mixture was diluted with a large amount of diethyl ether completing the precipitation of the compound XV, which was obtained in a pure form from iPrOH/$iPr_2O$ (1.38 g, 82% yield). $[\alpha]_D^{24}$ + 43.3°; $Rf_D$ 0.70; $E_{1.2}$ 0.71.

Example 6

Preparation of [H-Tyr-D-Ala-Phe-Gly-Tyr-Pro-Ser-NH]$_2$ • 2 HCl (XXII)

Step 1. [Z-Ser-NH]$_2$ (XVI)

Z-Ser-OH (2.39 g, 10 mmol) and Z-Ser-NH-$NH_2$ (2.90 g, 10 mmol) (J.S. Fruton, J. Biol. Chem. (1942) 146, 463) were coupled as described in the Example 4, Step 1, but purified as reported in the Example 1, Step 1, to give 3.65 g (77% yield) of pure compound XVI from AcOEt; $[\alpha]_D^{24}$ - 4.7°; $Rf_C$ 0.32.

Step 2. [H-Ser-NH]$_2$ • 2 HCl (XVII)

[Z-Ser-NH]$_2$ (3.52 g, 7.42 mmol) (XVI) was hydrogenated as described in the Example 5, Step 4, to give 1.68 g (81% yield) of compound XVII from iPrOH/$iPr_2O$; m.p. 230°C; $[\alpha]_D^{24}$ + 55.4° (c = 1 $H_2O$); $Rf_F$ 0.10; $E_{1.2}$ 2.13.

Step 3. [Boc-Tyr(Bzl)-Pro-Ser-NH]$_2$ (XVIII)

Boc-Tyr(Bzl)-Pro-OH (5.14 g, 10.96 mmol) (R. de Castiglione et al., Int. J. Pept. Protein Res. (1981) 17, 263) and [H-Ser-NH]$_2$ • 2 HCl (1.53 g, 4.58 mmol) (XVII) were condensed as described in the Example 1, Step 1, but purifying the product as described in the Example 5, Step 1, to give 5.04 g (83% yield) of compound XVIII from AcOEt/$Et_2O$; $Rf_C$ 0.29; $Rf_D$ 0.88.

Step 4. [H-Tyr(Bzl)-Pro-Ser-NH]$_2$ •2 HCl (XIX)

[Boc-Tyr(Bzl)-Pro-Ser-NH]$_2$ (4.82 g, 4.35 mmol) (XVIII) was deblocked as described in the Example 1, Step 2, to give 3.20 g (75% yield) of compound XIX from iPrOH/$Et_2O$; $Rf_D$ 0.21; $E_{1.2}$ 0.94.

Step 5. [Boc-Tyr-D-Ala-Phe-Gly-Tyr(Bzl)-Pro-Ser-NH}$_2$ (XX)

Boc-Tyr-D-Ala-Phe-Gly-OH (3.40 g, 6.10 mmol) (see Example 1, Step 1) and [H-Tyr(Bzl)-Pro-Ser-NH}$_2$ • 2 HCl (3.05 g, 3.11 mmol) (XIX) were condensed as described in Example 1, Step 1, to give 3.83 g (62% yield) of compound XX from iPrOH/iPr$_2$O; Rf$_C$ 0.12.

Step 6. [Boc-Tyr-D-Ala-Phe-Gly-Tyr-Pro-Ser-NH}$_2$ (XXI)

[Boc-Tyr-D-Ala-Phe-Gly-Tyr(Bzl)-Pro-Ser-NH}$_2$ (3.65 g, 1.84 mmol) (XX) was hydrogenated as described in Example 5, Step 4.

The reaction product was purified by FC eluting with $CH_2Cl_2$:MeOH = 9:1, to give 2.59 g (71% yield) of compound XXI from iPrOH/iPr$_2$O; Rf$_D$ 0.90; Rf$_G$ 0.34.

Step 7. [H-Tyr-D-Ala-Phe-Gly-Tyr-Pro-Ser-NH}$_2$ • 2 HCl (XXII)

[Boc-Tyr-D-Ala-Phe-Gly-Tyr-Pro-Ser-NH}$_2$ (2.40 g, 1.33 mmol) (XXI) was deblocked as described in the Example 1, Step 2. The reaction product was purified by gel chromatography on CM-Sephadex C-25, using as eluent system nBuOH:AcOH:$H_2O$ = 15:2:5. 1.12 g (50% yield) of compound XXII were obtained from iPrOH/iPr$_2$O; $[\alpha]_D^{24}$ - 1.1°; Rf$_D$ 0.49; $E_{1.2}$ 0.61.

Operating as described for the previous Examples the following other peptides have been synthesized:

- XXIV) [H-Tyr-D-Ala-Phe-Gly-NH}$_2$ • 2 HCl m.p. 160°-170°C (foam); $[\alpha]_D^{24}$ + 56.5°; Rf$_D$0.48; $E_{1.2}$0.79
- XXV) [H-Tyr-D-Ala-Phe-D-Ala-Tyr-NH}$_2$ • 2 HCl m.p. 222°-225°C (foam); $[\alpha]_D^{24}$ + 50.5°; $E_{1.2}$ 0.71
- XXVI) [H-Tyr-D-Arg-Phe-Sar-NH-$CH_2$}$_2$
- XXVII) [H-Tyr-D-Arg-Phe-Sar-Tyr-NH}$_2$
- XXVIII) [H-Tyr-D-Ala-Phe-Sar-NH-$CH_2$}$_2$ Rf$_D$ 0.50; $E_{1.2}$ 0.86
- XXIX) [H-Tyr-D-Ala-Phe-Sar-Tyr-NH}$_2$ Rf$_D$ 0.77; $E_{1.2}$ 0.71

**Claims**

**Claims for the following Contracting States : BE, CH, DE, FR, GB, IT, LI, LU, NL, SE**

**1.** A peptide of the formula I:

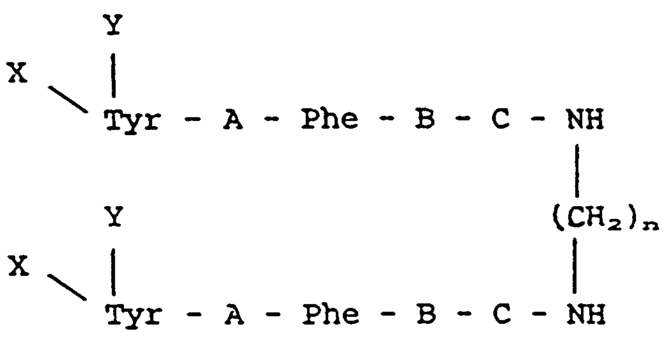

I

wherein

- X represents a hydrogen atom or a t-butyloxy carbonyl group,
- Y represents a hydrogen atom or a benzyl group
- A represents D-Ala or D-Arg residue,
- B represents a glycine, D-Ala or Sar residue,
- C represents a valence bond or Tyr, Tyr-Pro-Ser residue and
- n is 0, 2, 4 or 12; or the pharmaceutically acceptable salts thereof.

**2.** A compound of the formula:

```
H-Tyr-D-Ala-Phe-Gly-Tyr-NH
                        |
H-Tyr-D-Ala-Phe-Gly-Tyr-NH
```

or a pharmaceutically acceptable salt thereof.

**3.** A compound of the formula :

```
H-Tyr-D-Ala-Phe-Gly-Tyr-NH
                        |
                       (CH2)2
                        |
H-Tyr-D-Ala-Phe-Gly-Tyr-NH
```

or a pharmaceutically acceptable salt thereof.

**4.** A process for preparing a peptide of the formula I as defined in claim 1, which process comprises condensing a peptide of the formula II

```
X'  Y
  \ |
   Tyr - A - Phe - B - OH        II
```

wherein Y, A and B are as defined in claim 1 and X' has the same meanings of X stated in claim 1, but not hydrogen atom, with a compound of the formula III

$[H - C - NH - (CH_2)_n]_2$ III

wherein C and n are as defined above, using as activating method mixed anhydride, azide, activated ester or dicyclohexylcarbodiimide, optionally in the presence of a racemization preventing agent.

**5.** A process according to claim 4, wherein the condensation is carried out in dimethylformamide, dimethylacetamide, pyridine, acetonitrile, tetrahydrofuran or N-methyl-2-pyrrolidone, at a temperature of from -30 ° C to room temperature for a period of from 1 to 120 hours.

**6.** A pharmaceutical composition comprising as active ingredient a peptide or a pharmaceutically acceptable salt thereof as claimed in any one of claims 1 to 3 together with a pharmaceutically acceptable diluent or carrier.

**7.** A pharmaceutical composition according to claim 6 for extinguishing alcohol or opiate abstinence syndrome in mammals.

**8.** The use of a compound of formula I or a pharmaceutically acceptable salt thereof as claimed in any of claims 1 to 3 for the manufacture of a pharmaceutical composition for the treatment of addictive behaviour or opiate-dependence.

**Claims for the following Contracting State : AT**

**1.** The use of a peptide of the formula I:

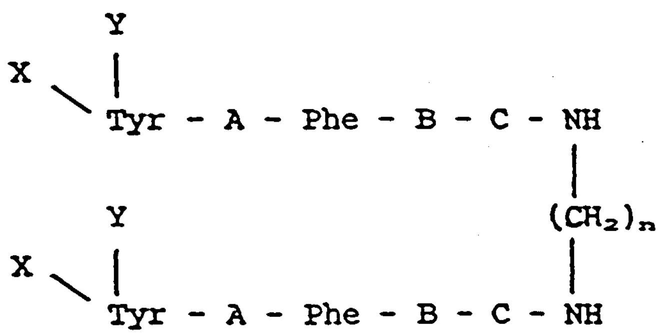

I

wherein

X represents a hydrogen atom or a t-butyloxy carbonyl group,
Y represents a hydrogen atom or a benzyl group
A represents D-Ala or D-Arg residue,
B represents a glycine, D-Ala or Sar residue,
C represents a valence hood or Tyr, Tyr-Pro-Ser residue and
n is 0, 2, 4 or 12; or the pharmaceutically acceptable salts thereof,

for the manufacture of a medicament for the treatment of addictive behaviour or opiate-dependence.

**2.** The use according to claim 1 wherein the peptide of general formula I has the formula:

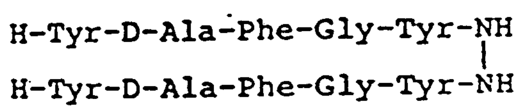

or a pharmaceutically acceptable salt thereof.

**3.** The use according to claim 1 wherein the peptide of general formula I has the formula:

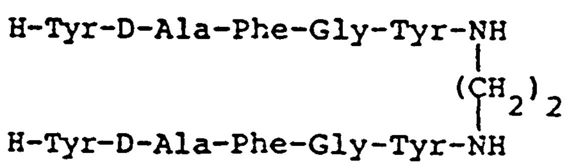

or a pharmaceutically acceptable salt thereof.

**4.** A process for preparing a peptide of the formula I as defined in claim 1, which process comprises condensing a peptide of the formula II

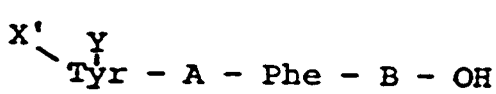

II

wherein Y, A and B are as defined in claim 1 and X' has the same meanings of X stated in claim 1, but not hydrogen atom, with a compound of the formula III

$[H - C - NH - (CH_2)_n]_2$ III

wherein C and n are as defined above, using as activating method mixed anhydride, azide, activated ester or dicyclohexylcarbodiimide, optionally in the presence of a racemization preventing agent.

**5.** A process according to claim 4, wherein the condensation is carried out in dimethylformamide, dimethylacetamide, pyridine, acetonitrile, tetrahydrofuran or N-methyl-2-pyrrolidone, at a temperature of from -30 °C to room temperature for a period of from 1 to 120 hours.

## Revendications

**Revendications pour les Etats contractants suivants : BE, CH, DE, FR, GB, IT, LI, LU, NL, SE**

**1.** Peptide représenté par la formule (I) :

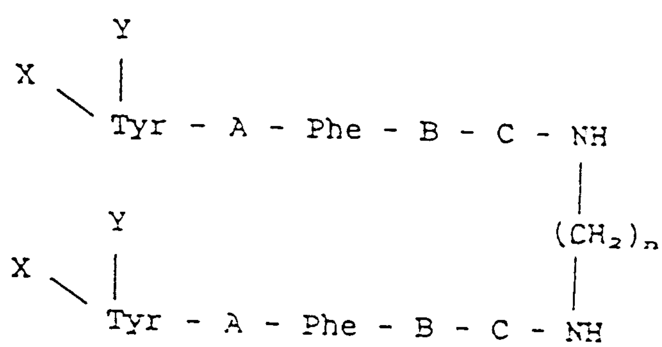

I

dans laquelle :

- X représente un atome d'hydrogène ou un groupe t-butyloxycarbonyle ;
- Y représente un atome d'hydrogène ou un groupe benzyle;
- A représente un reste D-Ala ou D-Arg ;
- B représente un reste glycine, D-Ala ou Sar ;
- C représente un liaison de valence ou un reste Tyr, Tyr-Pro-Ser ; et
- n vaut 0, 2, 4 ou 12 ;

ou les sels pharmaceutiquement acceptables dudit peptide.

**2.** Composé représenté par la formule :

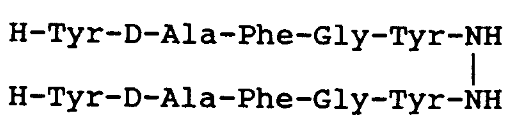

ou un sel pharmaceutiquement acceptable dudit composé.

3. Composé représenté par la formule :

```
H-Tyr-D-Ala-Phe-Gly-Tyr-NH
                         |
                        (CH2)2
                         |
H-Tyr-D-Ala-Phe-Gly-Tyr-NH
```

ou un sel pharmaceutiquement acceptable dudit composé.

4. Procédé de préparation d'un peptide représenté par la formule (I) telle que définie à la revendication 1, lequel procédé comprend la condensation d'un peptide représenté par la formule (II) :

```
X'   Y
  \  |
   Tyr - A - Phe - B - OH        II
```

dans laquelle :
- Y, A et B sont tels que définis à la revendication 1 ; et
- X' a les mêmes significations que X spécifié à la revendication 1, mais non un atome d'hydrogène,

avec un composé représenté par la formule (III) :

$[H - C - NH - (CH_2)_n]_2$ III

dans laquelle C et n sont tels que définis ci-dessus, en utilisant comme méthode d'activation, un anhydride mixte, azide, ester activé ou dicyclohexylcarbodiimide, facultativement en présence d'un agent empêchant la racémisation.

5. Procédé selon la revendication 4, dans lequel la condensation est effectuée dans le diméthylformamide, le diméthylacétamide, la pyridine, l'acétonitrile, le tétrahydrofuranne ou la N-méthyl-pyrrolidone-2, à une température de -30 °C à la température ambiante, pendant une période de temps de 1 à 120 heures.

6. Composition pharmaceutique comprenant, comme ingrédient actif, un peptide ou un sel pharmaceutiquement acceptable de celui-ci, tel que défini à l'une quelconque des revendications 1 à 3, conjointement avec un diluant ou support pharmaceutiquement acceptable.

7. Composition pharmaceutique selon la revendication 6 pour l'extinction du syndrome d'abstinence d'alcool ou d'opiacés chez les mammifères.

8. Utilisation d'un composé représenté par la formule (I) ou d'un sel pharmaceutiquement acceptable de ce composé, tel que défini à l'une quelconque des revendications 1 à 3, pour la fabrication d'une composition pharmaceutique pour le traitement du comportement d'addiction et de la dépendance vis-à-vis des opiacés.

**Revendications pour l'Etat contractant suivant: AT**

**1.** Utilisation d'un peptide représenté par la formule (I) :

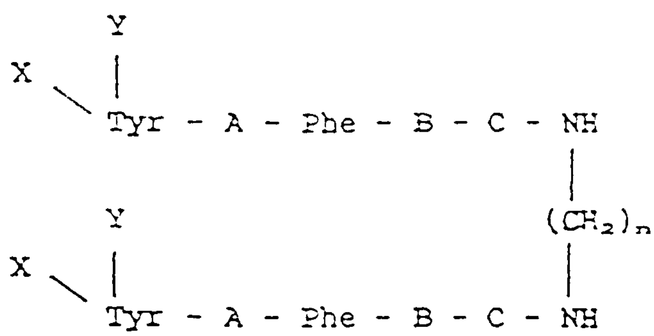

(I)

dans laquelle :

- X représente un atome d'hydrogène ou un groupe t-butyloxycarbonyle ;
- Y représente un atome d'hydrogène ou un groupe benzyle;
- A représente un reste D-Ala ou D-Arg ;
- B représente un reste glycine, D-Ala ou Sar ;
- C représente un liaison de valence ou un reste Tyr, Tyr-Pro-Ser ; et
- n vaut 0, 2, 4 ou 12 ;

  ou des sels pharmaceutiquement acceptables dudit peptide,

pour la fabrication d'un médicament pour le traitement du comportement d'addiction ou de la dépendance vis-à-vis des opiacés.

**2.** Utilisation selon la revendication 1, dans laquelle le peptide de formule générale (I) a la formule :

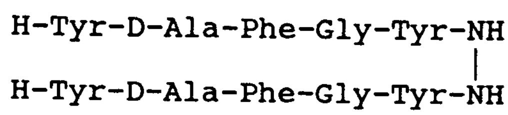

ou est un sel pharmaceutiquement acceptable de celui-ci.

**3.** Utilisation selon la revendication 1, dans laquelle le peptide de formule générale (I) a la formule :

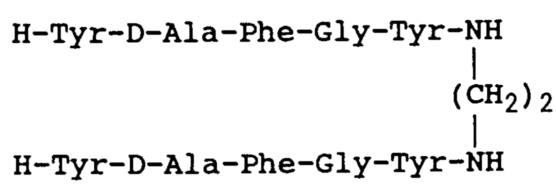

ou est un sel pharmaceutiquement acceptable de celui-ci.

**4.** Procédé de préparation d'un peptide représenté par la formule (I) telle que définie à la revendication 1, lequel procédé comprend la condensation d'un peptide représenté par la formule (II) :

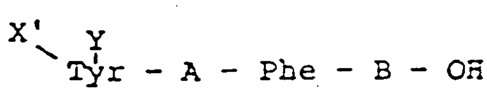

(II)

dans laquelle :

- Y, A et B sont tels que définis à la revendication 1 ; et
- X' a les mêmes significations que X spécifié à la revendication 1, mais non un atome d'hydrogène,

avec un composé représenté par la formule (III) :

$[H - C - NH - (CH_2)_n]_2$ (III)

dans laquelle C et n sont tels que définis ci-dessus, en utilisant comme méthode d'activation, un anhydride mixte, azide, ester activé ou dicyclohexylcarbodiimide, facultativement en présence d'un agent empêchant la racémisation.

**5.** Procédé selon la revendication 4, dans lequel la condensation est effectuée dans le diméthylformamide, le diméthylacétamide, la pyridine, l'acétonitrile, le tétrahydrofuranne ou la N-méthyl-pyrrolidone-2, à une température de -30 ° C à la température ambiante, pendant une période de temps de 1 à 120 heures.

**Patentansprüche**

**Patentansprüche für folgende Vertragsstaaten : BE, CH, DE, FR, GB, IT, LI, NL, SE**

**1.** Peptid der Formel I:

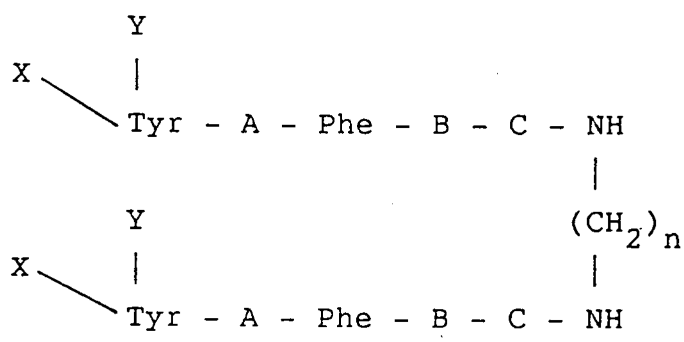

(I)

worin

X ein Wasserstoffatom oder eine t-Butyloxycarbonylgruppe darstellt,
Y stellt ein Wasserstoffatom oder eine Benzylgruppe dar,
A stellt einen D-Ala oder D-Arg Rest dar,
B stellt ein Glycin, einen D-Ala- oder Sar-Rest dar,
C stellt eine Valenzbindung oder einen Tyr-, Tyr-Pro-Ser-Rest dar und
n ist 0, 2, 4 oder 12;
oder pharmazeutisch annehmbare Salze davon.

**2.** Verbindung der Formel

H-Tyr-D-Ala-Phe-Gly-Tyr-NH
|
H-Tyr-D-Ala-Phe-Gly-Tyr-NH

oder ein pharmazeutisch annehmbares Salz davon.

**3.** Verbindung der Formel

```
H-Tyr-D-Ala-Phe-Gly-Tyr-NH
                         |
                      (CH2)2
                         |
H-Tyr-D-Ala-Phe-Gly-Tyr-NH
```

oder ein pharmazeutisch annehmbares Salz davon.

**4.** Verfahren zur Herstellung eines Peptids der Formel I wie in Anspruch 1 definiert, wobei das Verfahren die Kondensation eines Peptids der Formel II

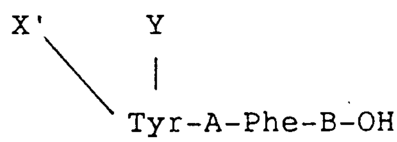

II

worin Y, A and B dieselbe Bedeutungen wie in Anspruch 1 haben und X' dieselbe Bedeutung wie X in Anspruch 1 hat, jedoch kein Wasserstoffatom ist, mit einer Verbindung der Formel III umfaßt

$[H\text{-}C\text{-}NH\text{-}(CH_2)_n\text{-}]\text{-}_2$ III

worin C und n dieselben Bedeutungen wie oben haben, unter Verwendung eines gemischten Anhydrids, Azids, aktivierten Esters oder Dicyclohexylcarbodiimids zur Aktivierung, wahlweise in Gegenwart eines Razemisierungsinhibitors.

**5.** Verfahren nach Anspruch 4, worin die Kondensation in Dimethylformamid, Dimethylacetamid, Pyridin, Acetonitril, Tetrahydrofuran oder N-Methyl-2-pyrrolidon bei einer Temperatur von -30 ° C bis Raumtemperatur in einem Zeitraum von 1 bis 120 Stunden durchgeführt wird.

**6.** Pharmazeutische Zusammensetzung enthaltend als wirksamen Bestandteil ein Peptid oder pharmazeutisch annehmbares Salz davon nach einem der Ansprüche 1 bis 3, zusammen mit einem pharmazeutisch annehmbaren Diluens oder Träger.

**7.** Pharmazeutische Zusammensetzung nach Anspruch 6 zur Beseitigung des Syndroms des Alkohol- oder Opiatentzugs bei Säugern.

**8.** Verwendung einer Verbindung der Formel I oder eines pharmazeutisch annehmbares Salzes davon nach einem der Ansprüche 1 bis 3 zur Herstellung einer pharmazeutischen Zusammensetzung zur Behandlung von Suchtverhalten oder Opiatabhängigkeit.

**Patentansprüche für folgenden Vertragsstaat : AT**

1. Verwendung eines Peptids der Formel I

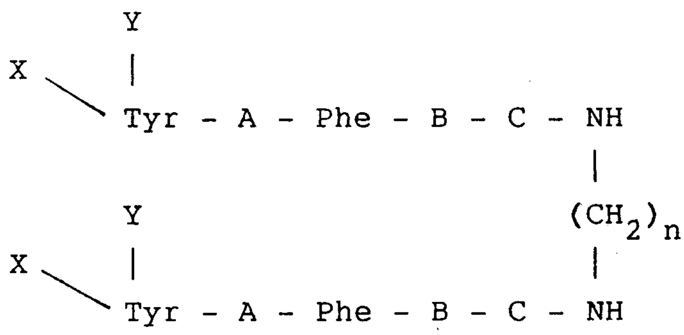

(I)

worin

X ein Wasserstoffatom oder eine t-Butyloxycarbonylgruppe darstellt,
Y stellt ein Wasserstoffatom oder eine Benzylgruppe dar,
A stellt einen D-Ala oder D-Arg Rest dar,
B stellt ein Glycin, einen D-Ala- oder Sar-Rest dar,
C stellt eine Valenzbindung oder einen Tyr-, Tyr-Pro-Ser-Rest dar und
n ist 0, 2, 4 oder 12;
oder pharmazeutisch annehmbare Salze davon,

zur Herstellung eines Medikaments zur Behandlung von Suchtverhalten oder Opiatabhängigkeit.

2. Verwendung nach Anspruch 1, worin das Peptid der allgemeinen Formel I die Formel hat

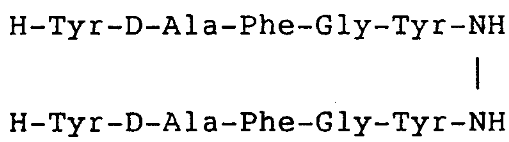

oder ein pharmazeutisch annehmbares Salz davon.

3. Verwendung nach Anspruch 1, worin das Peptid der allgemeinen Formel I die Formel hat

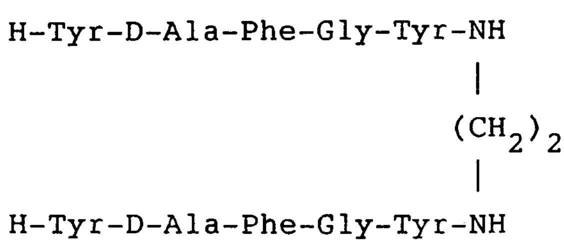

oder ein pharmazeutisch annehmbares Salz davon.

4. Verfahren zur Herstellung eines Peptids der Formel I wie in Anspruch 1 definiert, wobei das Verfahren die Kondensation eines Peptids der Formel II

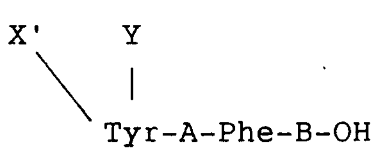

II

worin Y, A and B dieselbe Bedeutungen wie in Anspruch 1 haben und X' dieselbe Bedeutung wie X in Anspruch 1 hat, jedoch kein Wasserstoffatom ist, mit einer Verbindung der Formel III umfaßt

$[H\text{-}C\text{-}NH\text{-}(CH_2)_n\text{-}]\text{-}_2$ III

worin C und n dieselben Bedeutungen wie oben haben, unter Verwendung eines gemischten Anhydrids, Azids, aktivierten Esters oder Dicyclohexylcarbodiimids zur Aktivierung, wahlweise in Gegenwart eines Razemisierungsinhibitors.

**5.** Verfahren nach Anspruch 4, worin die Kondensation in Dimethylformamid, Dimethylacetamid, Pyridin, Acetonitril, Tetrahydrofuran oder N-Methyl-2-pyrrolidon bei einer Temperatur von -30 °C bis Raumtemperatur in einem Zeitraum von 1 bis 120 Stunden durchgeführt wird.